# EUROPÄISCHE PATENTSCHRIFT

(11) **EP 1 002 786 B1**
(45) Veröffentlichungstag und Bekanntmachung des Hinweises auf die Patenterteilung: **18.06.2003**
(21) Anmeldenummer: 99120451.2
(22) Anmeldetag: 14.10.1999
(51) Int. Cl.: C07C 51/347, C07C 51/38, C07C 63/04

(54) **Verfahren zur Herstellung von Trifluorbenzoesäuren**
Process for the preparation of trifluorobenzoic acids
Procédé pour la préparation d'acides trifluorobenzoiques

(30) Priorität: 04.11.1998 DE 19850788
(43) Veröffentlichungstag der Anmeldung: 24.05.2000
(73) Patentinhaber: Clariant GmbH, 65929 Frankfurt am Main (DE)
(72) Erfinder: Maier, Andreas, Dr., 65817 Eppstein (DE); Pfirmann, Ralf, Dr., 64347 Griesheim (DE)

(56) Entgegenhaltungen:
- EP-A- 0 287 951
- WO-A-89/06649
- CHEMICAL ABSTRACTS, vol. 117, no. 7, 17. August 1992 (1992-08-17) Columbus, Ohio, US; abstract no. 69742, MINAMIDA, AKIRA ET AL: "Preparation of 4-oxoquinoline-3-carboxylic acid derivatives as antibacterial agents" XP002130612 & JP 04 074167 A (DAINIPPON SEIYAKU K. K., JAPAN) 9. März 1992 (1992-03-09)

## Beschreibung

Die vorliegende Erfindung betrifft die Herstellung von Trifluorbenzoesäuren der Formel

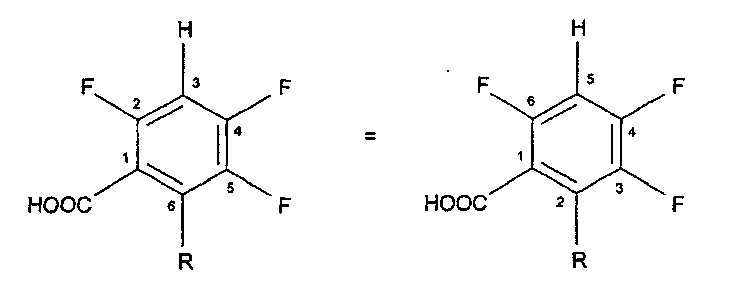

Derartige in 6-Stellung durch einen Rest R substituierte 2,4,5-Trifluorbenzoesäuren, die auch als in 2-Stellung substituierte 3,4,6-Trifluorbenzoesäuren bezeichnet werden können, stellen interessante Ausgangsverbindungen zur Herstellung von antibakteriell wirkenden Chinolon-Derivaten dar. H. Miyamoto et al. beschreiben in Bioorganic & Medicinal Chemistry 3 (1995) 1699 - 1706 ausgehend von 3,4,6-Trifluor-2-methylbenzoesäure (= 6-Methyl-2,4,5-trifluorbenzoesäure) die Herstellung von gegen grampositive Bakterien hochwirksamen 5-Methylchinoloncarbonsäurederivaten. Vergleiche auch Seite 1699, linke Spalte.

Die Herstellung der benötigten 3,4,6-Trifluor-2-methylbenzoesäure (6-Methyl-2,4,5-trifluorbenzoesäure) ist durch das nachfolgende Reaktionsschema in vereinfachter Form wiedergegeben. Vergleiche auch S. 1700 Herstellung der Verbindung 1 bis 4.

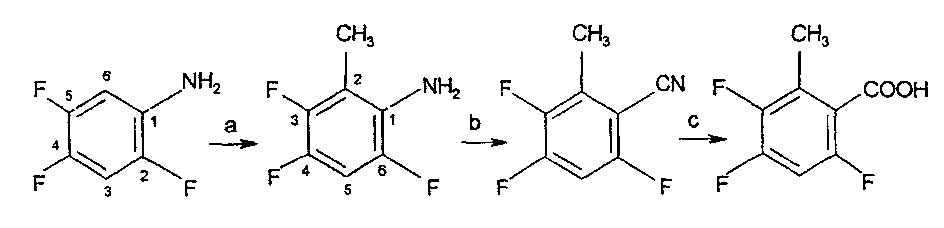

Die durch viele Einzelschritte charakterisierte, von 2,4,5-Trifluoranilin ausgehende Herstellung ist auf Seite 1703, linke Spalte in den die Herstellung von 3,4,6-Trifluor-2-methylanilin, 3,4,6-Trifluor-2-methylbenzonitril und 3,4,6-Trifluor-2-methylbenzoesäure (6-Methyl-2,4,5-trifluorbenzoesäure) betreffenden Beispielen beschrieben.
Die Nachteile dieser Herstellung bestehen darin, daß einerseits recht viele Einzelschritte erforderlich sind, unter anderem die nicht unproblematische Verwendung von Dimethylsulfid bei der Herstellung von 3,4,6-Trifluor-2-methylanilin, und andererseits das als Ausgangsmaterial verwendete 2,4,5-Trifluroanilin nicht leicht zugänglich ist, sondern nur über eine mehrstufige Synthese erhalten werden kann.

Von Nachteil ist ferner, daß die Ausbeute von 3,4,6-Trifluor-2-methylanilin lediglich 40 %, die von 3,4,6-Trifluor-2-methylbenzonitril lediglich 38 % und die von 3,4,6-Trifluor-2-methylbenzoesäure (6-Methyl-2,4,5-trifluorbenzoesäure) 79 % beträgt. Das entspricht einer Gesamtausbeute von nur 12 %, bezogen auf 2,4,5-Trifluoranilin. Diese sehr geringe Gesamtausbeute stellt keinen Anreiz dar, diese Art der Herstellung technisch zu realisieren.

Eine andere, von 1-Brom-2,4,5-trifluorbenzol ausgehende 4-stufige Herstellung, die durch das folgende Reaktionsschema vereinfacht dargestellt wird, ist J. Heterocycl. Chem. 27 (1990) 1609 - 1616 zu entnehmen

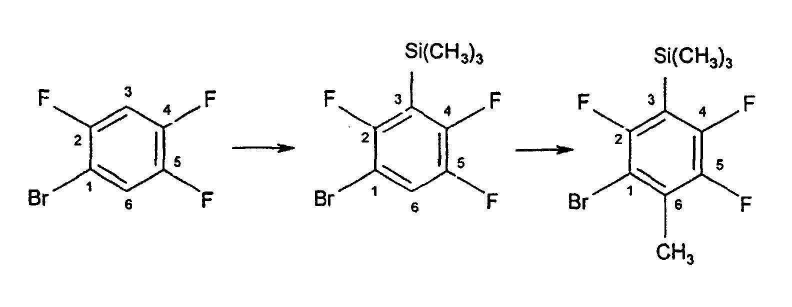

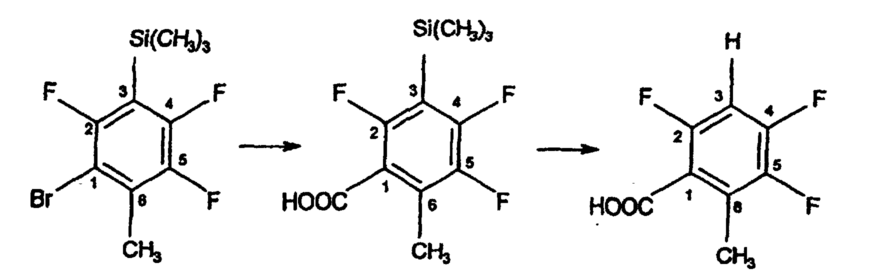

Diese Art der Herstellung ist sehr aufwendig. In den ersten und zweiten Syntheseschritten wird jeweils mit Butyllithium und Diisopropylamin in Tetrahydrofuran bei -78°C und im dritten Syntheseschritt mit Butyllithium in Ether ebenfalls bei -78°C gearbeitet. Im ersten Schritt wird 1-Brom-2,4,5-trifluorbenzol zunächst mit Lithiumdiisopropylamid und anschließend mit Trimethylsilylchlorid umgesetzt, das dabei erhaltene 1-Brom-2,4,5-trifluor-3-(trimethylsilyl)benzol (88 % Ausbeute) wind wiederum mit Lithiumdiisopropylamid und anschließend mit Trifluormethansäuremethylester umgesetzt, das dabei erhaltene 1-Brom-2,4,5-trifluor-6-methyl-3-(trimethylsilyl)benzol (75 % Ausbeute) wird mit Butyllithium und anschließend mit CO₂ in Form von Trockeneis umgesetzt und die dabei entstandene 2,4,5-Trifluor-6-methyl-3-(trimethylsilyl)benzoesäure (62 % Ausbeute) wird mit Cäsiumfluorid in Acetonitril zur 6-Methyl-2,4,5-trifluorbenzoesäure (89 % Ausbeute) umgesetzt.

Dieser Syntheseweg umfaßt viele Einzelschritte, verwendet zum Teil sehr teure Stoffe (z.B. Trifluormethansulfonsäuremethylester, Cäsiumfluorid) und liefert das Endprodukt in einer Gesamtausbeute von lediglich 36,4 %, bezogen auf 1-Brom-2,4,5-trifluorbenzot. Vergleiche auch die Beispiele in J. Heterocycl. Chem. 27 (1990) Seite 1612, rechte Spalte unten und Seite 1614 linke Spalte.

Verbindungen, wie z.B. 2,4,5-Trifluor-6-methylisophtalsäuredinitril und 2,4,5-Trifluor-6-methylisophthalsäure sind aus JP-A-4 074 167 bekannt.

Im Hinblick auf die vorstehend geschilderten Nachteile besteht die Aufgabe, ein Verfahren bereitzustellen, das diese Nachteile vermeidet, von gut zugänglichen Ausgangsstoffen ausgeht, sich mit vertretbarem Aufwand realisieren läßt und das gewünschte Endprodukt in akzeptabler Ausbeute zugänglich macht.

Diese Aufgabe wird gelöst durch ein Verfahren zur Herstellung von Trifluorbenzoesäuren der Formel 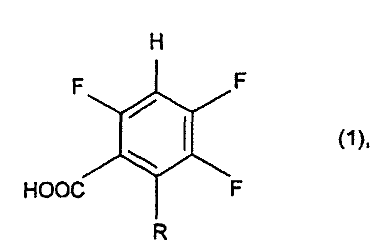 worin R für einen geradkettigen oder verzweigten Alkylrest mit 1 bis 6 C-Atomen, einen unsubstituierten Phenylrest, einen substituierten Phenylrest, der ein oder zwei Alkyl- oder Alkoxygruppen mit jeweils 1 bis 4 C-Atomen enthält, oder einen araliphatischen Rest mit 7 bis 12 Kohlenstoffatomen steht.
Es ist dadurch gekennzeichnet, daß man eine Verbindung der Formel 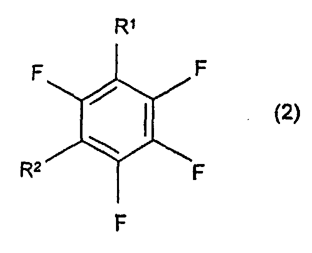 worin R¹ und R² gleich oder verschieden sind und für -CN, -COOR³, wobei R³ H, Li, Na, K, MgCl, MgBr, MgJ, ½Mg, ½Ca oder ein Alkylrest mit 1 bis 6 C-Atomen ist, oder -CONR⁴R⁵, wobei R⁴ und R⁵ gleich oder verschieden sind und einen Alkylrest mit 1 bis 6 C-Atomen bedeuten, stehen, mit wenigstens einer metallorganischen Verbindung der Formel CuR, CuLiR₂, MgXR, ZnR₂, LiR, AlX₂R, AlXR₂, AlR₃ oder Al₂Cl₃R₃, wobei R die in Formel (1) angegebene Bedeutung hat und X für Cl, Br oder J steht, in Gegenwart eines inerten Lösungsmittels bei -80 bis +150°C umsetzt, das Reaktionsprodukt in Gegenwart von Wasser in Abwesenheit oder Anwesenheit einer Säure bei 0 bis 250°C behandelt und die dabei entstehende Trifluorisophthalsäure der Formel 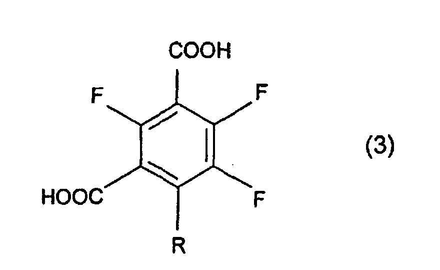 bei 80 bis 280°C decarboxyliert.

Die für das erfindungsgemäße Verfahren benötigten Ausgangsverbindungen der Formel (2) lassen sich - ausgehend von leicht zugänglichem Tetrachlorisophtalsäuredinitril, das durch Chlor-Fluor-Austausch (Umsetzung mit KF) leicht in das Tetrafluorisophthalsäuredinitril überführt werden kann, und erforderlichenfalls durch Umsetzung des Tetrafluorisophthalsäuredinitrils nach üblichen Methoden zu Tetrafluorisophthalsäure, Salzen der Tetrafluorisophthalsäure, Tetrafluorisophthalsäureestern und Tetrafluorisophthalsäureamiden - auf recht einfache Art und in guten Ausbeuten herstellen.

Das erfindungsgemäße Verfahren beruht auf einer kurzen Reaktionssequenz von lediglich drei Schritten, nämlich erstens auf der Umsetzung der Verbindung der Formel (2) mit der metallorganischen Verbindung, zweitens auf der Behandlung des dabei anfallenden Reaktionsproduktes mit der Säure in Gegenwart von Wasser und drittens auf der Decarboxylierung der Trifluorisophthalsäure, wobei das gewünschte Endprodukt der Formel (1), nämlich die in 6-Stellung substituierte 2,4,5-Trifluorbenzoesäure, erhalten wird.

Bei der Umsetzung von Carbonylgruppen mit metallorganischen Verbindungen, beispielsweise bei Umsetzung von Nitrilen oder Carbonsäureestern mit Grignard-Verbindungen, bilden sich üblicherweise Imine oder Amine respektive Ketone oder Alkohole. Es ist als überraschend anzusehen, daß diese Umsetzungen beim erfindungsgemäßen Verfahren nicht ablaufen.

Ein Austausch einer Nitrilgruppe gegen eine Alkylgruppe einer Grignard-Verbindung, wie er bei der Umsetzung von Tetrafluorterephthalsäurenitril mit Methylmagnesiumbromid unter Bildung von 4-Methyl-2,3,5,6-tetrafluorbenzonitril stattfindet (J. Organometallic Chem. 302 (1986), 147- 152), erfolgt nicht. Auch dies war nicht zu erwarten und ist ebenfalls als überraschend zu bewerten.

Ein weiterer Vorteil des erfindungsgemäßen Verfahrens besteht darin, daß im ersten Syntheseschritt der Austausch des Fluors in der 6-Stellung gegen den Rest R mit hoher Selektivität erfolgt und andere auf den Austausch anderer Fluorreste zurückgehende Isomere nur in recht geringem Umfange gebildet werden.

Es ist zudem überraschend, daß im letzten Syntheseschritt die Decarboxylierung der in 6-Stellung substituierten 2,4,5-Trifluorisophthalsäure mit sehr hoher Selektivität unter Bildung der entsprechenden in 6-Stellung substituierten 2,4,5-Trifluorbenzoesäure abläuft.

Man kann in das Verfahren mit gutem Erfolg eine Verbindung der Formel (2), worin R¹ und R² gleich oder verschieden sind und für -CN oder -COOR³ stehen, wobei R³ H, Li, Na, K, MgCl, MgBr, MgJ, ½Mg, ½Ca oder ein Alkylrest mit 1 bis 6, vorzugsweise 1 bis 4 Kohlenstoffatomen, insbesondere Na, K, MgCI oder MgBr, bevorzugt Na, K oder MgCl ist, einsetzen.

Es hat sich in einer Reihe von Fällen als günstig erwiesen, eine Verbindung der Formel (2), worin die Reste R¹ und R² gleich sind und die in Formel (2) angegebene Bedeutung haben, insbesondere gleich sind und für -CN, -COONa oder -COOMgCl, bevorzugt für -COONa oder -COOMgCI stehen, einzusetzen.

Es hat sich als vorteilhaft erwiesen, eine Verbindung der Formel (2), worin die Reste R¹ und R² gleich sind und für -COOLi, -COOMgCI, -COOMgBr oder -COOMgJ stehen, hergestellt durch Umsetzung von Tetrafluorisophthalsäure in einem inerten Lösungsmittel mit einer metallorganischen Verbindung LiR⁶, MgClR⁶, MgBrR⁶ oder MgJR⁶, worin R⁶ ein geradkettiger oder verzweigter Alkylrest mit 1 bis 6 Kohlenstoffatomen, ein unsubstituierter Phenylrest, ein substituierter Phenylrest, der ein oder zwei Alkyl- oder Alkoxygruppen mit jeweils 1 bis 4 C-Atomen enthält, oder ein araliphatischer Rest mit 7 bis 12 C-Atomen ist, einzusetzen.

Besonders vorteilhaft gestaltet sich das Verfahren, indem man eine Verbindung der Formel (2), worin R¹ und R² gleich sind und für -COOMgCI, -COOMgBr oder -COOMgJ, insbesondere -COOMgCI oder -COOMgBr, bevorzugt -COOMgCI stehen, hergestellt durch Umsetzung von Tetrafluorisophthalsäure in einem inerten Lösungsmittel mit MgClR⁶, MgBrR⁶ oder MgJR⁶, insbesondere MgClR⁶ oder MgBrR⁶, bevorzugt MgClR⁶, worin R⁶ für einen o-Tolylrest steht, einsetzt.

Man führt die Umsetzung der Tetrafluorisophthalsäure, die einer Verbindung der Formel (2), worin R¹ und R² jeweils -COOH sind, entspricht, üblicherweise unter den Bedingungen durch, die für die Umsetzung der Verbindung (2) mit der den Rest R enthaltenden metallorganischen Verbindung angewendet werden.

Man setzt die Tetrafluorisophthalsäure bei -80 bis +150°C, insbesondere -70 bis 100, bevorzugt 0 bis 80°C mit der den Rest R⁶ enthaltenden metallorganischen Verbindung um. Als inertes Lösungsmittel kann man eines der nachstehend noch näher beschriebenen inerten Lösungsmittel verwenden. Besonders einfach ist es, dasselbe Lösungsmittel, das bei der Umsetzung der Verbindung (2) mit der den Rest R enthaltenden metallorganischen Verbindung Anwendung findet, zu gebrauchen.

Wie zuvor erwähnt, setzt man wenigstens eine metallorganische Verbindung der Formel CuR, CuLiR₂, MgXR, ZnR₂, LiR, AlX₂R₁, AlXR₂, AlR₃ oder Al₂Cl₃R₃, insbesondere MgXR, LiR, AlX₂R, AlXR₂, AlR₃ oder Al₂Cl₃R₃, bevorzugt MgXR, LiR oder AlR₃ ein.

Man kann auch Mischungen der metallorganischen Verbindungen, beispielsweise (CuR + CuLiR), (MgCIR + MgBrR), (MgCIR + MgJR), (MgCIR + MgBrR + MgJR), (AlX₂R + AlXR₂), (AlX₂R + AlR₃) oder (AlXR₂ + AlR₃) verwenden.

In vielen Fällen genügt es, eine einzige metallorganische Verbindung der vorstehend genannten Art zu verwenden.

In der metallorganischen Verbindung hat der Rest R die in der Verbindung (1) angegebene Bedeutung. R steht insbesondere für einen geradkettigen oder verzweigten Alkylrest mit 1 bis 6, vorzugsweise 1 bis 4 C-Atomen, einen unsubstituierten Phenylrest oder einen substituierten Phenylrest, der ein oder zwei Alkyl- oder Alkoxygruppen enthält, bevorzugt für einen geradkettigen oder verzweigten Alkylrest mit 1 bis 6, vorzugsweise 1 bis 4 C-Atomen oder einen unsubstituierten Phenylrest, besonders bevorzugt für einen geradkettigen oder verzweigten Alkylrest mit 1 bis 6, vorzugsweise 1 bis 4 C-Atomen.

Die Umsetzung der Verbindung der Formel (2) mit der metallorganischen Verbindung erfolgt in Gegenwart eines inerten, das heißt unter den Bedingungen der Reaktion nicht reagierenden Lösungsmittels oder Lösungsmittelgemisches.

Man kann als inertes Lösungsmittel einen aromatischen Kohlenwasserstoff mit 6 bis 14, vorzugsweise 6 bis 10 C-Atomen, ein Trialkylamin mit 1 bis 18, vorzugsweise 2 bis 12 C-Atomen je Alkylrest, wobei der Alkylrest Alkoxygruppen mit 1 bis 4 C-Atomen als Substituenten enthalten kann, einen aliphatischen Ether mit 1 bis 6 C-Atomen je Alkylrest, einen cycloaliphatischen Ether mit 4 bis 6 Kohlenstoffatomen im Ring, einen Alkylether mehrwertiger Alkohole mit 2 bis 6 C-Atomen im Alkoholrest und 1 bis 4 C-Atomen je Alkylrest, einen Alkylether von Polyethylenglykol mit 2 bis 22, insbesondere 3 bis 12, bevorzugt 3 bis 8 Ethylenglykoleinheiten und 1 bis 4, insbesondere 1 bis 2 C-Atomen je Alkylrest oder ein Gemisch dieser Lösungsmittel, insbesondere einen aliphatischen Ether mit 1 bis 6, vorzugsweise 2 bis 4 C-Atomen je Alkylrest, einen cycloaliphatischen Ether mit 4 bis 6, vorzugsweise 4 bis 5 C-Atomen im Ring, einen Alkylether mehrwertiger Alkohole mit 2 bis 6, vorzugsweise 2 bis 4 C-Atomen im Alkoholrest und 1 bis 4, vorzugsweise 1 bis 2 C-Atomen, je Alkylrest einen Alkylether von Polyethylenglykol mit 2 bis 22, insbesondere 3 bis 12, bevorzugt 3 bis 8 Ethylenglykoleinheiten und 1 bis 4, insbesondere 1 bis 2 C-Atomen je Alkylrest oder ein Gemisch dieser Lösungsmittel einsetzen.

Als Beispiele für die vorstehend genannten inerten Lösungsmittel seien, ohne Anspruch auf Vollständigkeit zu erheben, Toluol, ortho-Xylol, meta-Xylol, para-Xylol, technische Gemische isomerer Xylole, Diphenylmethan, Trialkylamine mit 6 bis 14 Kohlenstoffatomen je Alkylrest, Gemische dieser Trialkylamine, beispielsweise mit 8 bis 10 Kohlenstoffatomen je Alkylrest (Hostarex A 327, Handelsprodukt der Hoechst AG), Methyl-tert.-butylether, Dimethoxyethan, Tetrahydrofuran, Dioxan, Diisopropylether, PEG-Dimethylether 250, Tributylamin, Diglyme, Triglyme, insbesondere Toluol, Tetrahydrofuran, Diglyme, Triglyme genannt. In vielen Fällen haben sich Toluol oder Tetrahydrofuran als besonders geeignet erwiesen.

Man führt die Umsetzung der Verbindung der Formel (2) mit der metallorganischen Verbindung wie zuvor bereits erwähnt bei -80 bis +150, insbesondere -70 bis + 100, bevorzugt -50 bis +70°C durch. Hierbei ist zu beachten, daß die zu wählende Temperatur in gewissem Umfange von den verwendeten metallorganischen Verbindungen und den Resten R¹ und R² in der Verbindung (2) abhängt. Metallorganische Verbindungen, die sehr reaktiv sind, erfordern in Verbindung mit aktivierenden Resten R¹ und R² vergleichsweise niedrige Temperaturen, beispielsweise -80 bis +50, insbesondere -50 bis +30°C, metallorganische Verbindungen mit mittlerer Reaktivität erfordern etwas höhere Temperaturen, beispielsweise -20 bis +100, insbesondere 0 bis 80°C, während metallorganische Verbindungen, die eine relativ geringe Reaktivität besitzen, bei vergleichsweise hohen Temperaturen, beispielsweise bei 50 bis 150, insbesondere 70 bis 140°C zum Einsatz gelangen.

Sehr reaktiv sind LiR, MgXR, CuLiR₂, eine mittlere Reaktivität besitzen ZnR₂, AlR₃, CuR während AlX₂R und AlXR₂ eine vergleichsweise niedrige Reaktivität zuzuordnen ist.

Aktivierende Gruppen R¹, R² sind -CN, -COOR³ mit R³ = Alkyl, weniger aktivierende Gruppen sind -CONR⁴R⁵; eine niedrige Reaktivität mit hoher Selektivität haben - COOR³ mit R³= H, Li, Na, K, MgCI, MgBr, MgJ, ½Mg und ½Ca.

Üblicherweise setzt man die Verbindung der Formel (2) in dem inerten Lösungsmittel in einer Konzentration von 1 bis 1000, insbesondere 10 bis 500, bevorzugt 50 bis 300 g je Liter inertes Lösungsmittel ein.

Man setzt üblicherweise die metallorganische Verbindung und die Verbindung der Formel (2) in einem Molverhältnis von (0,1 bis 5):1, insbesondere (0,2 bis 2): bevorzugt (0,3 bis 1,2):1, besonders bevorzugt (0,5 bis 1,1):1 ein.

In der Regel verwendet man auch die metallorganische Verbindung in Form einer Lösung, die üblicherweise 0,5 bis 10, insbesondere 1 bis 5, bevorzugt 1,5 bis 4 mol metallorganische Verbindung pro Liter inertes Lösungsmittel enthält.

Man kann die erforderliche Menge der Verbindung (2) ganz oder teilweise vorlegen und anschließend bei Reaktionstemperatur die metallorganische Verbindung unter Rühren zusetzen. Es ist auch möglich, die Verbindung (2) und die metallorganische Verbindung getrennt voneinander, aber gleichzeitig zuzugeben und unter Rühren umzusetzen.

Nach Beendigung der Umsetzung rührt man noch kurze Zeit nach, zersetzt eventuell noch vorhandene metallorganische Verbindung mit Wasser, entfernt das inerte Lösungsmittel und behandelt das durch Umsetzung der Verbindung der Formel (2) mit der metallorganischen Verbindung entstehende Reaktionsprodukt in Gegenwart von Wasser in Abwesenheit oder Anwesenheit einer Säure, insbesondere mit einer wäßrigen Säure. Man kann als Säure eine Mineralsäure oder eine aliphatische Carbonsäure mit 1 bis 4 Kohlenstoffatomen, insbesondere eine Halogenwasserstoffsäure, Phosphorsäure, Salpetersäure, Schwefelsäure, Essigsäure oder ein Gemisch, bevorzugt Schwefelsäure verwenden.

In einer Reihe von Fällen kann es günstig sein, das Reaktionsprodukt vor der Behandlung mit Säure destillativ zu reinigen und nicht umgesetztes Ausgangsmaterial zurückzugewinnen.

Man führt die Behandlung mit Säure bei 0 bis 250°C durch. Enthält das Reaktionsprodukt -CN, -COOR³ (R³ = Alkyl) oder -CONR⁴R⁵ als Reste, so führt man die Behandlung mit der Säure bei Temperaturen von 50 bis 250°C, insbesondere 100 bis 200°C, bevorzugt 120 bis 180°C durch und überführt diese Reste in die entsprechende Carbonsäure.

Enthält das Reaktionsprodukt -COOLi, -COONa, -COOK, -COOMgCI, -COOMgBr, -COOMgJ, -COOMg/2 oder -COOCa/2 als Reste, so genügt üblicherweise ein relativ geringer stöchiometrischer Säureüberschuß von 1 bis 100, insbesondere 5 bis 20, bevorzugt 10 bis 15 % bei Temperaturen von 0 bis 50°C, um die -COOH Gruppe entstehen zu lassen. Hohe Temperaturen sind hierbei nicht erforderlich.

Man setzt das durch Umsetzung der Verbindung der Formel (2), worin R¹, R², -CN, -CONR⁴R⁵, -COOR³ (R³ = Alkyl) ist, mit der metallorganischen Verbindung entstehende Reaktionsprodukt mit Wasser, bezogen auf die Verbindung der Formel (2), üblicherweise im Gewichtsverhältnis 100:1 bis 0,5:1, insbesondere 10:1 bis 1:1 um. Die Säure wird hierbei, bezogen auf eingesetztes Wasser, üblicherweise im Gewichtsverhältnis 0,001:1 bis 10:1, insbesondere 0,01:1 bis 5:1, bevorzugt 0,1:1 bis 2:1 eingesetzt.
Durch die Behandlung mit Säure in Gegenwart von Wasser bildet sich durch Hydrolyse die Trifluorisophthalsäure der Formel

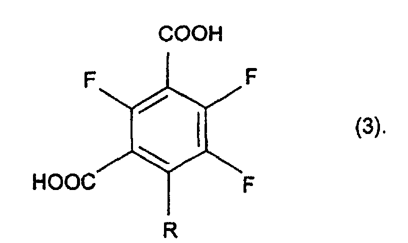

Aus dem die Trifluorisophthalsäure der Formel (3) enthaltenden Reaktionsgemisch wird, gegebenenfalls nach Verdünnen mit Wasser, die Trifluorisophthalsäure durch Extraktion mittels eines mit Wasser nicht mischbaren Lösungsmittel gewonnen.

Anstelle der Extraktion kann man das die Trifluorisophthalsäure der Formel (3) enthaltende Reaktionsgemisch durch Destillation in Abwesenheit oder Anwesenheit eines hochsiedenden Lösungsmittel einengen, die Trifluorisophthalsäure der Formel (3) abfiltrieren und anschließend decarboxylieren oder die Trifluorisophthalsäure der Formel (3) in Gegenwart des hochsiedenden Lösungsmittel decarboxylieren.

Als mit Wasser nicht mischbares Lösungsmittel kann beispielsweise eines der vorstehend aufgeführten inerten Lösungsmittel- oder Lösungsmittelgemische - soweit nicht mit Wasser mischbar oder in Wasser löslich - für die Extraktion der Trifluorisophthalsäure verwendet werden.

Ohne Anspruch auf Vollständigkeit zu erheben, seien als mit Wasser nicht mischbare Lösungsmittel, Dialkylether mit 1 bis 6, insbesondere 2 bis 4 Kohlenstoffatomen je Alkylrest, aromatische Kohlenwasserstoffe mit 6 bis 12, insbesondere 6 bis 9 Kohlenstoffatomen, einfach oder mehrfach chlorierte aromatische Kohlenwasserstoffe mit 6 bis 12, insbesondere 6 bis 9 Kohlenstoffatome, aliphatische Kohlenwasserstoffe mit 5 bis 12 Kohlenstoffatomen, einfach oder mehrfach chlorierte aliphatische Kohlenwasserstoffe mit 1 bis 8, insbesondere 1 bis 6 Kohlenstoffatomen, cycloaliphatische Kohlenwasserstoffe mit 5 bis 12, insbesondere 6 bis 9 Kohlenstoffatomen, einfach oder mehrfach chlorierte cycloaliphatische Kohlenwasserstoffe mit 5 bis 12, insbesondere 6 bis 9 Kohlenstoffatomen, aliphatische Ketone mit 4 bis 12, insbesondere 5 bis 10 Kohlenstoffatomen, Ester aliphatischer Carbonsäuren mit 1 bis 6 Kohlenstoffatomen und aliphatischer Alkohole mit 1 bis 4 Kohlenstoffatomen oder Gemische dieser Lösungsmittel genannt.

Beispiele für geeignete mit Wasser nicht mischbare Lösungsmittel sind Diethylether, Methyl-tert.-butylether, Dibutylether, Benzol, Toluol, ortho-Xylol, meta-Xylol, para-Xylol, Gemische isomerer Xylole, Chlorbenzol, Dichlorbenzol, Chlortoluol, Dichlortoluol, Hexan, Dichlormethylen, Chloroform, Trichlorethan, Cyclohexan, Methylethylketon, Diethylketon, Methylpropylketon, Methylbutylketon, Di-n-butylketon, Methylacetat, Ethylacetat, Propylacetat, Butylacetat, Methylpropionsäureester, Methylbuttersäureester oder Mischungen dieser Lösungsmittel.

Falls gewünscht, kann nach Entfernen des Lösungsmittel zur Reinigung umkristallisiert werden. Geeignete Lösungsmittel hierfür sind beispielsweise Wasser, wäßrige Säuren, insbesondere wäßrige Mineralsäuren, Essigsäure, aliphatische Alkohole mit 1 bis 4 C-Atomen und aliphatische Kohlenwasserstoffe mit 5 bis 12 C-Atomen.

Besonders gut geeignet sind Wasser, wäßrige Säuren und Hexan.

Die Decarboxylierung wird in Gegenwart eines inerten Lösungsmittels oder einer Base, insbesondere einer N-enthaltenden organischen Verbindung oder in Gegenwart eines inerten Lösungsmittels und einer Base, insbesondere einer N-enthaltenden organischen Verbindung, durchgeführt.

Die Decarboxylierung der Trifluorisophthalsäure der Formel (3) erfolgt üblicherweise durch Erhitzen in einem inerten Lösungsmittel. Als inertes Lösungsmittel kann man einen aliphatischen Kohlenwasserstoff mit 8 bis 20 C-Atomen, einen aromatischen Kohlenwasserstoff mit 6 bis 15 C-Atomen, einen chlorierten aliphatischen Kohlenwasserstoff mit 8 bis 20 C-Atomen, einen chlorierten aromatischen Kohlenwasserstoff mit 6 bis 15 C-Atomen, ein Dialkylamid einer aliphatischen Carbonsäure mit 1 bis 6 C-Atomen und eines Dialkylamins mit 1 bis 4 Kohlenstoffatomen, beispielsweise Dimethylformamid, Diethylformamid, Dimethylacetamid, Diethylacetamid, N-Methylpyrrolidon, Tetramethylharnstoff, ein aliphatisches Sulfon, beispielsweise Dimethylsulfoxid oder Sulfolan, ein aliphatisches oder aromatisches Nitril, beispielsweise Acetonitril oder Benzonitril, einen aliphatischen Ether mit 4 bis 10 C-Atomen je Alkylrest, einen Alkylether mehrwertiger Alkohole mit 2 bis 6, vorzugsweise 2 bis 4 C-Atomen im Alkoholrest und 1 bis 4, vorzugsweise 1 bis 2 C-Atomen je Alkylrest, einen Alkylether von Polyethylenglykol mit 2 bis 22, insbesondere 3 bis 12, bevorzugt 3 bis 8 Ethylenglykoleinheiten und 1 bis 4, insbesondere 1 bis 2, C-Atomen je Alkylrest, beispielsweise Triglyme oder PEG 250, einen aliphatischen Alkohol mit 4 bis 10 C-Atomen, ein aliphatisches Diol mit 2 bis 6, beispielsweise Ethylenglykol, ein aliphatisches Triol, beispielsweise Glycerin, einsetzen.
In einer Vielzahl von Fällen hat es sich als günstig erwiesen als inertes Lösungsmittel Sulfolan, Dimethylformamid, Dimethylacetamid oder Triglyme einzusetzen.

Die für die Decarboxylierung erforderliche Reaktionstemperatur beträgt - wie eingangs schon erwähnt 80 bis 280°C. In vielen Fällen hat sich eine Reaktionstemperatur von 100 bis 270, insbesondere 120 bis 250, bevorzugt 120 bis 230°C als für die Durchführung der Decarboxylierung ausreichend erwiesen.

Man kann die Decarboxylierung in Gegenwart einer in Wasser löslichen Base oder einer mit Wasser nicht löslichen organischen Base, insbesondere in Gegenwart einer mit Wasser nicht löslichen organischen Base durchführen.

Als organische Base kommen N-enthaltende organische Verbindungen, insbesondere ein in Wasser nichtlösliches Amin in Betracht.

Die in Wasser lösliche oder in Wasser unlösliche organische Base, insbesondere das in Wasser nichtlösliche Amin kann in vergleichsweise geringen Mengen, aber auch in relativ hohen Mengen Anwendung finden. Üblicherweise setzt man je Mol der Trifluorisophthalsäure der Formel (3) 0,001 bis 50, insbesondere 0,01 bis 2, bevorzugt 0,05 bis 1, besonders bevorzugt 0,1 bis 0,5 Mol der organischen Base, insbesondere des in Wasser nichtlöslichen Amins ein. Die organische Base kann auch alleine ohne Zusatz eines der vorstehenden inerten Lösungsmittel eingesetzt werden. Man kann aber auch die Decarboxylierung in Gegenwart sowohl eines inerten Lösungsmittels als auch einer Base durchführen.

Unter dem Begriff in Wasser nichtlösliches Amin werden solche Amine verstanden, die sich entweder in Wasser nur in geringem Umfange oder gar nicht lösen. Üblicherweise verwendet man als in Wasser nichtlösliches Amin ein Alkylamin mit 6 bis 30 Kohlenstoffatomen, ein Dialkylamin mit 6 bis 30 Kohlenstoffatomen je Alkylrest, ein Trialkylamin mit 4 bis 30 Kohlenstoffatomen je Alkylrest, eine N-enthaltende heterocyclische Verbindung oder ein Gemisch der vorstehend genannten Stoffe, insbesondere ein Alkylamin mit 8 bis 20 Kohlenstoffatomen im Alkylrest, ein Dialkylamin mit 8 bis 20 Kohlenstoffatomen je Alkylrest, ein Trialkylamin mit 6 bis 20 Kohlenstoffatomen je Alkylrest, ein gegebenenfalls alkyliertes Chinolin oder Pyridin, beispielsweise Collidin, Lutidin oder Picolin oder ein Gemisch der vorstehend genannten Stoffe, bevorzugt ein Trialkylamin mit 6 bis 20, insbesondere 6 bis 14, bevorzugt 8 bis 12 Kohlenstoffatomen je Alkylrest oder ein Gemisch dieser Trialkylamine.

Ohne Anspruch auf Vollständigkeit zu erheben, seien als Beispiele für geeignete Amine genannt: n-Hexylamin, Isohexylamin, n-Heptylamin, Isoheptylamin, n-Octylamin, Isooctylamin, n-Nonylamin, Isononylamin, n-Decylamin, Isodecylamin, n-Dodecylamin, Isododecylamin, n-Hexadecylamin, Isohexadecylamin, Di-n-hexylamin, Diisohexylamin, Di-n-heptylamin, Diisoheptylamin, Di-n-octylamin, Diisooctylamin, Di-n-nonylamin, Diisononylamin, Di-n-decylamin, Diisodecylamin, Di-n-dodecylamin, Diisododecylamin, Di-n-hexadecylamin, Diisohexadecylamin, Tri-n-hexylamin, Tri-isohexylamin, Tri-n-heptylamin, Triisoheptylamin, Tri-n-octylamin, Triisoctylamin, Tri-n-decylamin, Triisodecylamin, Tri-n-dodecylamin, Triisododecylamin, Trialkylamine mit geradkettigen und/oder verzweigten Ketten mit 6 bis 14 Kohlenstoffatomen, Pyridin, α-Picolin, β-Picolin, γ-Picolin, 2,4-Dimethylpyridin(α,γ-Lutidin), 2,6-Di-tert.-butylpyridin, 2,4,6-Trimethylpyridin (Collidin), Triethylpyridin, Chinolin, Methylchinoline, Ethylchinoline, gemischte Amine wie Butyldihexylamin, Dioctyldecylamin, Hexyldioctylamin, Dihexyloctylamin, Diheptyloctylamin, Didecyloctylamin, Didodecyloctylamin, Didodecyldecylamin, Didecyldodecylamin, Dioctyldodecylamin, Dinonyloctylamin, Dinonyldecylamin, Dinonyldodecylamin.

Es lassen sich generell auch beliebige Mischungen der vorstehend genannten, in Wasser nicht löslichen Amine, insbesondere Gemische verschiedener Alkyl-, Dialkylund Trialkylamine, vorzugsweise Gemische verschiedener Trialkylamine mit 6 bis 14, insbesondere 8 bis 12 Kohlenstoffatomen einsetzen.

Die Decarboxylierung läßt sich in Anwesenheit oder Abwesenheit eines üblichen Decarboxylierungskatalysators durchführen. Geeignet als Decarboxylierungskatalysator ist Kupfer, eine Kupfer(I)verbindung oder eine Kupfer(II)verbindung, beispielsweise Kupfer(I)oxid, Kupfer(II)oxid, Kupfer(I)sulfat, Kupfer(II)sulfat, Kupfer(I)chlorid, Kupfer(II)chlorid, Kupfer(I)fluorid, Kupfer(II)fluorid, Kupfercarbonat, Kupfer(I)hydroxid, Kupfer(II)hydroxid, bevorzugt Kupfer(I)oxid und Kupfer(II)oxid. Es lassen sich auch beliebige Mischungen der vorstehend genannten Stoffe verwenden.

Üblicherweise setzt man den Decarboxylierungskatalysator in einer Menge von 0,1 bis 10, insbesondere 0,5 bis 3 Mol-%, bezogen auf die Trifluorisophthalsäure der Formel (3), ein.

Bei Verwendung eines wasserunlöslichen inerten Lösungsmittels extrahiert man nach Beendigung der Decarboxylierung mit einer wäßrigen Lauge, beispielsweise Natronlauge oder Kalilauge, und erhält einen basischen Extrakt des Reaktionsproduktes.

Bei Verwendung eines wasserlöslichen inerten Lösungsmittels säuert man nach Beendigung der Decarboxylierung mit wäßriger Säure an und extrahiert dann das Reaktionsprodukt mittels eines wasserunlöslichen Lösungsmittels.

Aus diesem Extrakt wird mittels einer wäßrigen Base das Salz der Trifluorbenzoesäure der Formel (1) reextrahiert und anschließend als basischer Extrakt abgetrennt. Die basischen Extrakte werden angesäuert und die durch das Ansäuern freigesetzte Trifluorbenzoesäure der Formel (1) kristallisiert oder wird mittels eines in Wasser nicht löslichen organischen Lösungsmittel extrahiert. Aus der organischen Phase kann die Trifluorbenzoesäure durch Abdampfen des Lösungsmittels oder durch Kristallisation gewonnen werden. Falls gewünscht, kann die Trifluorbenzoesäure durch Umkristallisieren, zum Beispiel aus Wasser oder wäßriger Säure oder Hexan weiter gereinigt werden. In der Regel fällt die Trifluorbenzoesäure bereits in sehr hoher Reinheit an, so daß eine zusätzliche Reinigung nicht erforderlich ist.

Das Verfahren läßt sich kontinuierlich oder diskontinuierlich ausführen. Man kann bei Unterdruck, Normaldruck oder Überdruck arbeiten.

Die nachfolgenden Beispiele beschreiben die vorliegende Erfindung näher, ohne sie hierauf zu beschränken.

### Experimenteller Teil

### Herstellung der Ausgangsverbindungen

Die folgenden Versuche A und B sind nicht Gegenstand der vorliegenden Erfindung, sondern dienen zur Demonstration der einfachen Herstellbarkeit der Ausgangsverbindungen.

### Versuch A: Herstellung von Tetrafluorisophthaiodinitril

200 g Tetrachlorisophthalodinitril, 250 g Kaliumfluorid, 16 g Tetraphenylphosphoniumbromid, 250 g Toluol werden in 750 g Sulfolan vorgelegt, dann destilliert man 245 g Destillat ab und erhöht die Temperatur auf 215 °C, nach 3 Stunden wird abgekühlt, nach der Aufarbeitung bestehend aus Filtration und Destillation des Reaktionsgemisches erhält man: 112 g Tetrafluorisophthalodinitril (Sdp. 148-151°C bei 7 mbar, Reingehalt > 98 % nach GC). Das entspricht 75 % Ausbeute.
Schmp.: 77 bis 78°C

### Versuch B: Herstellung von Tetrafluorisophthalsäure

20 g Tetrafluorisophthalodinitril werden in 80 g 70 %iger Schwefelsäure 30 min. lang auf 160°C erhitzt, dann wird abgekühlt, auf 240 g Eis gegossen und mit 80 g Methylisobutylketon extrahiert. Nach Entfernen des Lösungsmittels verbleiben 22,8 g Tetrafluorisophthalsäure (Reinheit nach HPLC > 99 %ig). Das entspricht einer Ausbeute von 95,7 %.
Schmp.: 216 °C (Messung mit DSC), Lit.: 212 bis 215°C

Die nachfolgenden Beispiele dienen zur Erläuterung des erfindungsgemäßen Verfahrens:

### Herstellung von 2,4,5-Trifluor-6-methylisophthalsäuredinitril

### Beispiel 1a: Überschuß an Methylmagnesiumbromid

In einem 100 ml Mehrhalskolben werden unter Schutzgas 5,04 g (25,2 mmol) Tetrafluorisophthalodinitril gelöst in 50 ml Tetrahydrofuran vorgelegt und auf -50°C gekühlt. Dann tropft man bei dieser Temperatur 11,3 ml einer 3M Lösung von Methylmagnesiumbromid (34 mmol) innerhalb einer Stunde zu. Nach 1 Stunde Nachrühren bei -50°C wird das Reaktionsgemisch in 20 ml Wasser gegossen und mit Salzsäure ein pH von 1 eingestellt. Die Reaktionsmischung wird mit Dichlormethan extrahiert und das Lösungsmittel entfernt. Als Rückstand verbleiben 6,3 g eines hellgelben Öls. Die GC Analyse dieses Reaktionsprodukts zeigt folgende Zusammensetzung:
< 1 % (GC-FID) Tetrafluoroisophthalodinitril
58,7 % (GC-FID) 2,4,5-Trifluor-6-methylisophthalsäuredinitril (Selektivität 58 %, Umsatz > 99 %)
41,3 % (GC-FID) Nebenprodukte

Das Reaktionsprodukt enthält nach HPLC (w/w): 43 Gew.-% 2,4,5-Trifluor-6-methylisophthalsäuredinitril; dies entspricht einer Ausbeute von 55 %.

### Beispiel 1b: Unterschuß an Methylmagnesiumbormid

Es wird, wie in Beispiel 1 angegeben, verfahren, jedoch werden 2 g (10 mmol) Tetrafluorisophthalodinitril und 3 ml Methylmagnesiumbromidlösung (9mmol) eingesetzt und 2,1 g eines Rohprodukts mit folgender Zusammensetzung erhalten:
42,2 % (GC-FID) Tetrafluorisophthalodinitril
53,6 % (GC-FID) 2,4,5-Trifluor-6-methylisophthalsäuredinitril (Selektivität nach GC 93 %, Umsatz 58 %)
4,1 % (GC-FID) Nebenprodukte,

### Herstellung von 2,4,5-Trifluor-6-methylbenzoesäure aus Tetrafluorisophthalodinitril

### Beispiel 2:

### a) Herstellung von 2,4,5-Trifluor-6-methylisophthalsäuredinitril

250 g Tetrafluorisophtalodinitril werden in 2,5 l Tetrahydrofuran gelöst und bei -50°C vorgelegt. Dann tropft man bei dieser Temperatur über 8 Stunden 420 ml einer 3 M Lösung von Methylmagnesiumchlorid zu. Der Ansatz wird in Wasser (5 l) gegeben und mit insgesamt 1 l Dichlormethan extrahiert, nach Entfernen des Lösungsmittels werden 320,3 g Reaktionsprodukt erhalten. Die Fraktionierung liefert: 97,4 g 2,4,5-Trifluor-6-methylisophthalsäuredinitril (Reingehalt 97 % nach GC) und 87 g Tetrafluorisophtalodinitril (RG > 98 % nach GC), die für den Folgeansatz wieder eingesetzt werden. Aus dem Folgeansatz werden in analoger Vorgehensweise 33,5 g 2,4,5-Trifluor-6-methylisophthalsäuredinitril (Reingehalt 97 % nach GC FID) und 29,3 g Tetrafluorisophtalodinitril erhalten, das bei nochmaliger Verwendung 11 g 2,4,5-Trifluor-6-methylisophthalsäuredinitril liefert.

Die Gesamtausbeute dieser drei Umsetzungen beträgt somit 137,7 g = 56,2 % bezogen auf ursprünglich eingesetztes Tetrafluorisophthalodinitril.

2,4,5-Trifluor-6-methylisophthalsäuredinitril ist durch folgende Daten charakterisiert: 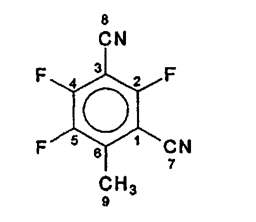 GC/MS: m/z = 196

| ¹H-NMR, 400.13 MHz, DMSO-d₆, (TMS) = 0 | | | | | |
|---|---|---|---|---|---|
| Signal | Multiplizität | (¹H) | Anzahl H | *J*_{F,H} [Hz] | Zuordnung |
| a | d | 2.56 | 3 | *J*_{5-F,H} 2.6 | C*H*₃ |

| ¹³C-NMR, 100.61 MHz, DMSO-d₆, (DMSO-d₆) = 39.5 | | | | | |
|---|---|---|---|---|---|
| Signal | Multiplizität¹ | (¹³C) | Anzahl C | *J*_{F,C} [Hz] | Zuordnung |
| A | s | 159.48 | 1 | ¹*J*_{2-F,C} = 264.8, 4.3, 3.2 | C-2 |
| B | s | 153.05 | 1 | ¹*J*_{4-F,C} = 267.8, ²*J*_{5-F,C} = 17.0, 5.7 | C-4 |
| C | s | 145.01 | 1 | ¹*J*_{5-F,C} = 245.8, ²*J*_{4-F,C} = 11.4, 3.9 | C-5 |
| D | s | 138.58 | 1 | ²*J*_{5-F,C} = 18.1, 2.9, 1.7 | C-6 |
| E | s | 110.50 | 1 | 2.9, 2.2 | C-7 o.C-8 |
| F | s | 107.63 | 1 | 3.3 | C-7 o. C-8 |
| G | s | 99.73 | 1 | ²*J*_{2-F,C} = 15.5, 5.0, 3.2 | C-1 |
| H | s | 92.19 | 1 | ²*J*_{F,C} = 19.8, ²*J*_{F,C} = 16.8, 2.1 | C-3 |
| I | q | 14.09 | 1 | 2.7, 2.7, 1.8 | *C*H₃ |

| | | | | | |
|---|---|---|---|---|---|
| ¹ ohne Berücksichtigung der durch J _{F,C} verursachten Signalaufspaltungen | | | | | |

| ¹⁹F-NMR, 376.50 MHz, DMSO-d₆, [CFCl₃ (virt. int.)] = 0 | | | | | | |
|---|---|---|---|---|---|---|
| Signal | Multiplizität² | δ(¹⁹F) | Anzahl F | *J*_{F,F}[HZ] | ¹*J*_{F,C} [Hz]³ | Zuordnung |
| α | d | -104.2 | 1 | ⁵*J*_{2-F,5-F} = 13.8 | 264.9 | 2-F |
| β | d | -119.4 | 1 | ³*J*_{4-F,5-F} = 21.8 | 267.9 | 4-F |
| γ | dd | -139.2 | 1 | | 245.9 | 5-F |

| | | | | | | |
|---|---|---|---|---|---|---|
| ¹ ohne Berücksichtigung der durch J _{F,C} verursachten Signalaufspaltungen | | | | | | |
| ² bezogen auf ¹⁹F {¹H} | | | | | | |
| ³ aus den ¹³C-Sateliten | | | | | | |

### b) Herstellung von 2,4,5-Trifluor-6-methylbenzoesäure

3,0 g des oben hergestellten 2,4,5-Trifluor-6-methylisophthalsäuredinitrils werden in 20 ml 70 %iger Schwefelsäure 24 Stunden lang auf 130°C erhitzt. Nach dem Abkühlen wird der Ansatz in 80 ml Eiswasser gegeben und mit Methylisobutylketon extrahiert. Nach Entfernen des Lösungsmittels wird ein gelber Feststoff (3,4 g) erhalten, der nach HPLC eine Reinheit > 95 % aufweist und mit dem in Beispiel 3 beschriebenen Reaktionsprodukt identisch ist.

Ohne weitere Reinigung wird dieser Feststoff zusammen mit 10 g Hostarex A 327 (Gemisch von Trialkylaminen mit 8 bis 10 C-Atomen je Alkylrest; Handelsprodukt der Hoechst AG) 2 Stunden lang auf 150°C erhitzt, die Aufarbeitung erfolgt analog zu Beispiel 4. Man erhält 2,4 g 2,4,5-Trifluor-6-methylbenzoesäure (Reingehalt nach HPLC 97 %), was einer Ausbeute von 82 %, bezogen auf eingesetztes 2,5,6-Trifluor-4-methylisophthalodinitril, und 46 %, bezogen auf eingesetztes Tetrafluorisophthalodinitril, entspricht.

### Herstellung von 2,4,5-Trifluor-6-methylisophthalsäure aus Tetrafluorisophthalsäure

### Beispiel 3:

4 g Tetrafluorisophthalsäure werden unter Schutzgas in 50 ml Tetrahydrofuran bei 20°C vorgelegt, dann werden innerhalb von 1 Stunde 11,2 ml einer 3 molaren Lösung von Methylmagnesiumchlorid zugetropft, um das Chloromagnesiumsalz der Tetrafluorisophthalsäure zu bilden.

Zu der erhaltenen Suspension werden bei 20 bis 30°C weitere 5,7 ml einer 3 molaren Lösung von Methylmagnesiumchlorid gegeben. Nach beendeter Zugabe wird noch 1 Stunde lang nachgerührt, dann mit 100 g Wasser hydrolysiert und mit Salzsäure angesäuert. Extraktion mit Methylisobutylketon und Entfernen des Lösungsmittels liefert 3,8 g eines Reaktionsprodukts, das nach NMR-Spektroskopie 85 % 2,4,5-Trifluor-6-methylisophthalsäure und 11% unumgesetzte Tetrafluorisophthalsäure enthält. Daraus ergibt sich eine Selektivität von 95 % und eine Ausbeute von 82 %. Die HPLC-Reinheit des Rohprodukts beträgt 79 %. Die Reinigung erfolgt durch Kristallisation aus verdünnter Salzsäure.

2,4,5-Trifluor-6-methylisophthalsäure ist durch folgende Daten charakterisiert: 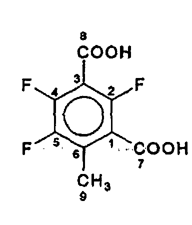 Schmp.: 254°C (aus verdünnter Salzsäure, Messung mit DSC)

| ¹H-NMR, 400.13 MHz, DMSO-d₆, δ(DMSO-d₆) = 2.50 | | | | | |
|---|---|---|---|---|---|
| Signal | Multiplizität | δ (¹H) | Anzahl H | *J*_{F,H} [Hz] | Zuordnung |
| a | br. s | 14,02 | 2 | | O*H* |
| b | d | 2,31 | 3 | *J*_{5-F,H} = 2,4 | C*H*₃ |

| ¹³C-NMR, 100.61 MHz, DMSO-d₆, δ(DMSO-d₆) = 39.5 | | | | | |
|---|---|---|---|---|---|
| Signal | Multiplizität⁴ | δ (¹³C) | Anzahl C | *J*_{F,C} [Hz] | Zuordnung |
| A | s | 164.17 | 1 | *J*_{F,C} = 2.1, 0.8 | C-7 o. C-8 |
| B | s | 160.76 | 1 | *J*_{F,C} = 2.4, > 1.1, > 0.5 | C-8 o. C-7 |
| C | s | 151.50 | 1 | ¹*J*_{F,C} = 252.7, *J*_{F,C} = 6.3, 3.2 | C-2 |
| D | s | 147.74 | 1 | ¹*J*_{4-F,C} = 256.8, ²*J*_{5-F,C} = 16.1, ³*J*_{2-F,C} = 8.2 | C-4 |
| E | s | 145.04 | 1 | ¹*J*_{5-F,C} = 243.2, ²*J*_{4-F,C} = 12.9, ⁴*J*_{2-F,C} = 3.7 | C-5 |
| F | s | 128.30 | 1 | ²*J*_{F,C} = 16.6, *J*_{F,C} = 4.8, 1.4 | C-6? |
| G | s | 120.35 | 1 | ²*J*_{F,C} = 19.7, *J*_{F,C} = 4.1, 2.2 | C-1? |
| H | s | 111.31 | 1 | ^{2*J*}_{F,C} = 22.1, 16.2 | C-3 |
| I | q | 11.95 | 1 | > 1.9 | *C*H₃ |

| | | | | | |
|---|---|---|---|---|---|
| ⁴ ohne Berücksichtigung der durch J_{F,C} verursachten Signalaufspaltungen | | | | | |

| ¹⁹F-NMR, 376.50 MHz, DMSO-d₆, δ[CFCl₃ (virt. int.)] = 0 | | | | | | |
|---|---|---|---|---|---|---|
| Signal | Multiplizität⁵ | δ (¹⁹F) | Anzahl F | *J*_{F,F}[Hz] | ¹*J*_{F,C} [Hz]⁶ | Zuordnung |
| α | dd | -118.4 | 1 | ⁵*J*_{2-F,5-F} = 15.2, ⁴*J*_{2-F,4-F} = 1.3 | 252.8 | 2-F |
| β | dd | -133.6 | 1 | ³*J*_{4-F,5-F} = 22.3 | 257.3 | 4-F |
| γ | dd | -142.9 | 1 | | 243.0 | 5-F |

| | | | | | | |
|---|---|---|---|---|---|---|
| ⁵ bezogen auf ¹⁹F {1H} | | | | | | |
| ⁶ aus den ¹³C-Satelliten | | | | | | |

### Herstellung von 2,4,5-Trifluor-6-methylbenzoesäure aus Tetrafluorisophthalsäure

### Beispiel 4:

20 g Tetrafluorisophthalsäure werden in 250 ml THF vorgelegt und bei 20 bis 40°C mit 88 ml einer 3 molaren Methylmagnesiumchloridlösung versetzt. Nach der üblichen wäßrigen Aufarbeitung (siehe Beispiel 3) erhält man 20,1 g 2,4,5-Trifluor-6-methylisophthalsäure. Die HPLC-Reinheit des Produkts beträgt 85 %.

Ohne weitere Reinigung werden 20 g dieses Rohprodukts in 80 g Hostarex A 327 suspendiert und 2 Stunden lang auf 150°C erhitzt. Nach dem Abkühlen wird alkalisch mit 80 g Wasser und 8 g 50 %iger Natronlauge extrahiert, die wäßrige Produktphase mit Salzsäure angesäuert und der entstandene Niederschlag abfiltriert. Es werden 14,8 g Filterkuchen erhalten. Trocknung bei 50°C ergibt 13,8 g 2,4,5-Trifluor-6-methylbenzoesäure (Reinheit nach HPLC 90 %). Nach Kristallisation dieses Rohprodukts aus Wasser werden 11,4 g 2,4,5-Trifluor-6-methylbenzoesäure erhalten, Reingehalt nach HPLC 99,5 %, was einer Ausbeute von 71 %, bezogen auf eingesetzte Tetrafluorisophthalsäure, entspricht.

2,4,5-Trifluor-6-methylbenzoesäure ist durch folgende Daten charakterisiert. 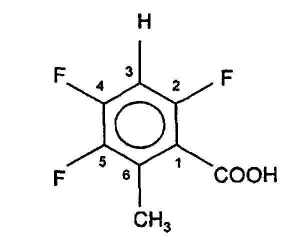 Schmp.: 121°C (aus Wasser, Messung mit DSC) Lit.: 116 bis 117°C

| ¹H-NMR, 400.13 MHz, DMSO-d₆, δ(TMS) = 0 | | | | | |
|---|---|---|---|---|---|
| Signal | Multiplizität | δ (¹H) | Anzahl H | *J*_{F,H} [Hz] | Zuordnung |
| a | "dt" | 7.47 | 1 | ³*J*_{4-F,H}⁷= 10.6, ³*J*_{2-F,H}⁷= 9.5, ⁴*J*_{5-F,H} = 3.6? | 3-H |
| b | d | 2.32 | 3 | *J*_{5-F,H} = 2.6 | C*H*₃ |

| | | | | | |
|---|---|---|---|---|---|
| ⁷ aus ¹H gekoppeltem ¹⁹F-NMR-Spektrum | | | | | |

| ¹³C-NMR, 100.61 MHz, DMSO-d₆, δ(DMSO-d₆) = 39.5 | | | | | |
|---|---|---|---|---|---|
| Signal | Multiplizität⁸ | δ (¹³C) | Anzahl C | *J*_{F,C} [Hz] | Zuordnung |
| A | s | 164.64 | 1 | > 2.1, 0.8 | *C*OOH |
| B | s | 154.23 | 1 | ¹*J*_{F,C} = 247.0, 11.6, 3.1 | C-2 |
| C | s | 149.97 | 1 | ¹*J*_{F,C} = 250.7, 15.1, 14.0 | C-4 |
| D | s | 144.89 | 1 | ¹*J*_{F,C} = 241.1, ²*J*_{F,C} = 12.6, 3.8 | C-5 |
| E | s | 126.33 | 1 | ²*J*_{F,C} = 16.5, 4.6, 1.2 | C-6? |
| F | s | 119.79 | 1 | ²*J*_{F,C} = 18.9, 4.0, 2.2 | C-1? |
| G | d | 103.89 | 1 | ²*J*_{F,C} = 28.7 u. 21.7 | C-3 |
| H | q | 11.71 | 1 | 4.2, 2.5, 1.8 | *C*H₃ |

| | | | | | |
|---|---|---|---|---|---|
| ⁸ ohne Berücksichtigung der durch J_{F,C} verursachten Signalaufspaltungen | | | | | |

| ¹⁹F-NMR, 376.50 MHZ, DMSO-d₆, δ[CFCl₃ (virt. int.)] = 0 | | | | | | |
|---|---|---|---|---|---|---|
| Signal | Multiplizität⁹ | δ (¹⁹F) | Anzahl F | *J*_{F,F}[Hz] | ¹*J*_{F,C} [Hz]¹⁰ | Zuordnung |
| α | dd | -116.0 | 1 | ⁵*J*_{2-F,5-F} = 15.0, ⁴*J*_{2-F,4-F} = 5.0 | 246.9 | 2-F |
| β | dd | -131.9 | 1 | ³*J*_{4-F,5-F} = 22.3 | 250.5 | 4-F |
| γ | dd | -144.9 | 1 | | 241.1 | 5-F |

| | | | | | | |
|---|---|---|---|---|---|---|
| ⁹ bezogen auf ¹⁹F {¹H} | | | | | | |
| ¹⁰ aus den ¹³C-Satelliten | | | | | | |

### Herstellung von 2,4,5-Trifluor-6-methylbenzoesäure aus Tetrafluorisophthalsäure

### Beispiel 4A:

36 g Tetrafluorisophthalsäure werden unter Schutzgas in 500 ml Tetrahydrofuran bei 20°C vorgelegt. Dann werden 160 ml einer 1,7 molaren Lösung von ortho-Tolylmagnesiumchlorid in THF zugetropft und 1 Stunde nachgerührt, um das Chloromagnesiumsalz der Tetrafluorisophthalsäure zu bilden.

Dann werden 76 ml einer 3 molaren Methylmagnesiumchloridlösung in THF innerhalb von 2 Stunden zudosiert und 30 min nachgerührt.

Nach Aufarbeitung wie in Beispiel 3 werden 40,4 g Rohprodukt mit einer HPLC Reinheit von 73 % erhalten.

Ohne weitere Reinigung werden 40 g dieses Rohprodukts analog Beispiel 4 in Hostarex A 327 eingetragen und 2 Stunden auf 150°C erhitzt. Nach der Aufarbeitung erhält man 44 g Rohprodukt (HPLC-Reinheit 87 %), durch Umlösen in Wasser werden daraus 16,5 g 2,4,5-Trifluor-6-methylbenzoesäure erhalten (Ausbeute 57,5 % bezogen auf eingesetzte Tetrafluorisophthalsäure).

### Herstellung von 2,4,5-Trifluor-6-methylbenzoesäure durch Decarboxylierung von 2,4,5-Trifluor-6-methylisophthalsäure

### Beispiele 5 bis 8: Orientierende Versuche bezüglich der einsetzbaren Lösungsmittel

Jeweils 0,5 g 6-Methyl-2,4,5-trifluorisophthalsäure werden in 5 g Lösungsmittel erhitzt und per HPLC wird der Umsatz zu 2,4,5-Trifluor-6-methylbenzoesäure bestimmt. Die folgende Tabelle faßt die Ergebnisse zusammen:

| Beispiel | Lösungsmittel | Temperatur | Zeit | Umsatz nach HPLC (a/a) |
|---|---|---|---|---|
| 5 | Sulfolan | 200°C | 8 h | 38 % |
| 6 | Sulfolan | 220°C | 4 h | 99 % |
| 7 | Triglyme | 215 °C | 6 h | 99 % |
| 8 | Hostarex A327 | 150 °C | 2 h | 99% |

### Hostarex A 327 ist ein Gemisch von Trialkylaminen mit 8 bis 10 C-Atomen je Alkylrest

### Beispiel 9: Präparative Umsetzung in Sulfolan

18 g 2,4,5-Trifluor-6-methylisophthalsäure (90 %ig) werden in Sulfolan bei 220°C 5 Stunden lang erhitzt. Nach dem Abkühlen und der Zugabe von 400 g Wasser wird mit konzentrierter Salzsäure sauer gestellt und dann mit 80 g Toluol extrahiert. Der Toluolextrakt wird mit 50 g Wasser versetzt, mit Natronlauge alkalisch gestellt und die Phasen getrennt. Durch Ansäuern wird das Produkt (16,1 g) aus der Wasserphase freigesetzt. Es fällt als erstarrendes Öl an, das bei 50°C abgetrennt wird und nach HPLC eine Reinheit von 89 % aufweist. Nach NMRspektroskopischen Untersuchungen enthält dieses Produkt noch 6 mol% Sulfolan.

## Patentansprüche

1. Verfahren zur Herstellung von Trifluorbenzoesäuren der Formel 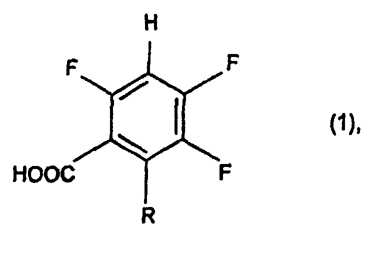 worin R für einen geradkettigen oder verzweigten Alkylrest mit 1 bis 6 C-Atomen, einen unsubstituierten Phenylrest, einen substituierten Phenylrest, der ein oder zwei Alkyl- oder Alkoxygruppen mit jeweils 1 bis 4 C-Atomen enthält, oder einen araliphatischen Rest mit 7 bis 12 C-Atomen steht, **dadurch gekennzeichnet, daß** man eine Verbindung der Formel 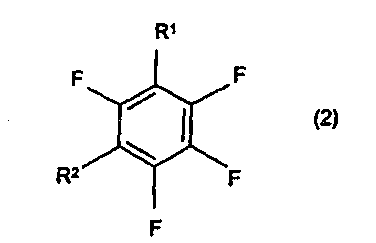 worin R¹ und R² gleich oder verschieden sind und für -CN, -COOR³, wobei R³ H, Li, Na, K, MgCl, MgBr, MgJ, ½Mg, ½Ca oder ein Alkylrest mit 1 bis 6 C-Atomen ist, oder -CONR⁴R⁵, wobei R⁴ und R⁵ gleich oder verschieden sind und einen Alkylrest mit 1 bis 6 C-Atomen bedeuten, stehen, mit wenigstens einer metallorganischen Verbindung der Formel CuR, CuLiR₂, MgXR, ZnR₂, LiR, AlX₂R, AlXR₂, AlR₃ oder Al₂Cl₃R₃, wobei R die in Formel (1) angegebene Bedeutung hat und X für Cl, Br oder J steht, in Gegenwart eines inerten Lösungsmittels bei -80 bis +150°C umsetzt, das Reaktionsprodukt in Gegenwart von Wasser in Abwesenheit oder Anwesenheit einer Säure bei 0 bis 250°C behandelt und die dabei entstehende Trifluorisophthalsäure 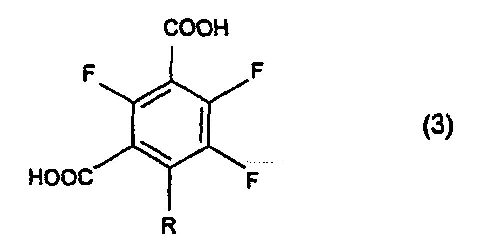 bei 80 bis 280°C decarboxyliert.

2. Verfahren nach Anspruch 1, **dadurch gekennzeichnet, daß** man eine Verbindung der Formel (2), worin R¹ und R² gleich oder verschieden sind und für -CN oder -COOR³, wobei R³ Na, K, MgCl oder MgBr ist, stehen, einsetzt.

3. Verfahren nach Anspruch 1 oder 2, **dadurch gekennzeichnet, daß** man eine Verbindung der Formel (2), worin R¹ und R² gleich sind und für -CN, -COONa oder -COOMgCl stehen, einsetzt.

4. Verfahren nach einem oder mehreren der Ansprüche 1 bis 3, **dadurch gekennzeichnet, daß** man eine Verbindung der Formel (2), worin R¹ und R² gleich sind und für -COOLi, -COOMgCl, -COOMgBr oder -COOMgJ stehen, hergestellt durch Umsetzung von Tetrafluorisophthalsäure in einem inerten Lösungsmittel mit einer metallorganischen Verbindung LiR⁶, MgClR⁶ oder MgJR⁶, worin R⁶ ein geradkettiger oder verzweigter Alkylrest mit 1 bis 6 Kohlenstoffatomen, ein unsubstituierter Phenylrest, ein substituierter Phenylrest, der ein oder zwei Alkyloder Alkoxygruppen mit jeweils 1 bis 4 C-Atomen enthält, oder ein araliphatischer Rest mit 7 bis 12 C-Atomen ist, einsetzt.

5. Verfahren nach einem oder mehreren der Ansprüche 1 bis 4, **dadurch gekennzeichnet, daß** man eine Verbindung der Formel (2), worin R¹ und R² gleich sind und für -COOMgCl, -COOMgBr oder -COOMgJ stehen, hergestellt durch Umsetzung von Tetrafluorisophthalsäure in einem inerten Lösungsmittel mit einer metallorganischen Verbindung MgClR⁶, MgBrR⁶ oder MgJR⁶, worin R⁶ für einen o-Tolylrest steht, einsetzt.

6. Verfahren nach einem oder mehreren der Ansprüche 1 bis 5, **dadurch gekennzeichnet, daß** man eine metallorganische Verbindung der Formel MgXR, LiR, AlX₂R, AlXR₂, AlR₃ oder Al₂Cl₃R₃ einsetzt.

7. Verfahren nach einem oder mehreren der Ansprüche 1 bis 6, **dadurch gekennzeichnet, daß** man eine metallorganische Verbindung der Formel MgXR, LiR oder AlR₃ einsetzt.

8. Verfahren nach einem oder mehreren der Ansprüche 1 bis 7, **dadurch gekennzeichnet, daß** man als inertes Lösungsmittel einen aromatischen Kohlenwasserstoff mit 6 bis 14 C-Atomen, ein Trialkylamin mit 1 bis 18 C-Atomen je Alkylrest, wobei der Alkylrest Alkoxygruppen mit 1 bis 4 C-Atomen als Substituenten enthalten kann, einen aliphatischen Ether mit 1 bis 6 C-Atomen je Alkylrest, einen cycloaliphatischen Ether mit 4 bis 6 Kohlenstoffatomen im Ring, einen Alkylether mehrwertiger Alkohole mit 2 bis 6 C-Atomen im Alkoholrest und 1 bis 4 C-Atomen je Alkylrest, einen Alkylether von Polyethylenglykol mit 2 bis 22 Ethylenglykoleinheiten und 1 bis 4 C-Atomen je Alkylrest oder ein Gemisch dieser Lösungsmittel einsetzt.

9. Verfahren nach einem oder mehreren der Ansprüche 1 bis 8, **dadurch gekennzeichnet, daß** man als inertes Lösungsmittel einen aliphatischen Ether mit 1 bis 6 C-Atomen je Alkylrest, einen cycloaliphatischen Ether mit 4 bis 6 C-Atomen im Ring, einen Alkylether mehrwertiger Alkohole mit 2 bis 6 C-Atomen im Alkoholrest und 1 bis 4 C-Atomen je Alkylrest oder ein Gemisch dieser Lösungsmittel einsetzt.

10. Verfahren nach einem oder mehreren der Ansprüche 1 bis 9, **dadurch gekennzeichnet, daß** man bei -70 bis +100°C umsetzt.

11. Verfahren nach einem oder mehreren der Ansprüche 1 bis 10, **dadurch gekennzeichnet, daß** man das Reaktionsprodukt mit einer Mineralsäure oder einer aliphatischen Carbonsäure mit 1 bis 4 Kohlenstoffatomen als Säure behandelt.

12. Verfahren nach einem oder mehreren der Ansprüche 1 bis 11, **dadurch gekennzeichnet, daß** man das Reaktionsprodukt mit Schwefelsäure als Säure behandelt.

13. Verfahren nach einem oder mehreren der Ansprüche 1 bis 12, **dadurch gekennzeichnet, daß** man in Gegenwart eines inerten Lösungsmittels oder einer Base oder in Gegenwart eines inerten Lösungsmittels und einer Base decarboxyliert.

## Claims

1. A process for preparing trifluorobenzoic acids of the formula 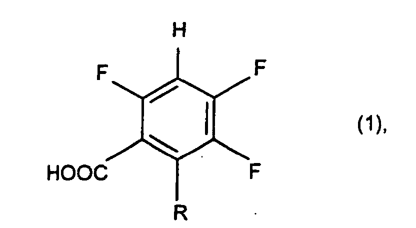 in which R is a straight-chain or branched alkyl radical having 1 to 6 C atoms, an unsubstituted phenyl radical, a substituted phenyl radical which contains one or two alkyl or alkoxy groups having in each case 1 to 4 C atoms, or an araliphatic radical having 7 to 12 C atoms, which comprises reacting a compound of the formula 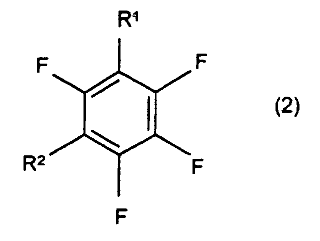 in which R¹ and R² are identical or different and are -CN, -COOR³, where R³ is H, Li, Na, K, MgCl, MgBr, MgI, ½Mg, ½Ca or an alkyl radical having 1 to 6 C atoms, or -CONR⁴R⁵, where R⁴ and R⁵ are identical or different and are an alkyl radical having 1 to 6 C atoms, with at least one organometallic compound of the formula CuR, CuLiR₂, MgXR, ZnR₂, LiR, AlX₂R, AlXR₂, AlR₃ or Al₂Cl₃R₃, where R is as defined in formula (1) and X is Cl, Br or I, in the presence of an inert solvent at from -80 to +150°C, treating the reaction product in the presence of water in the absence or presence of an acid at from 0 to 250°C and decarboxylating the resulting trifluoroisophthalic acid of the formula 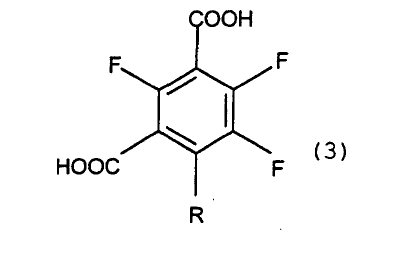 at from 80 to 280°C.

2. The process as claimed in claim 1, wherein a compound of the formula (2) in which R¹ and R² are identical or different and are -CN or -COOR³, where R³ is Na, K, MgCl or MgBr, is used as starting material.

3. The process as claimed in claim 1 or 2, wherein a compound of the formula (2) in which R¹ and R² are identical and are -CN, -COONa or -COOMgCl is used as starting material.

4. The process as claimed in one or more of claims 1 to 3, wherein a compound of the formula (2) in which R¹ and R² are identical and are -COOLi, -COOMgCl, -COOMgBr or -COOMgI, prepared by reaction of tetrafluoroisophthalic acid in an inert solvent with an organometallic compound LiR⁶, MgClR⁶ or MgIR⁶, in which R⁶ is a straight-chain or branched alkyl radical having 1 to 6 carbon atoms, an unsubstituted phenyl radical, a substituted phenyl radical which contains one or two alkyl or alkoxy groups having in each case 1 to 4 C atoms, or an araliphatic radical having 7 to 12 C atoms, is used as starting material.

5. The process as claimed in one or more of claims 1 to 4, wherein a compound of the formula (2) in which R¹ and R² are identical and are -COOMgCl, -COOMgBr or -COOMgI, prepared by reaction of tetrafluoroisophthalic acid in an inert solvent with an organometallic compound MgClR⁶, MgBrR⁶ or MgIR⁶, in which R⁶ is an o-tolyl radical, is used as starting material.

6. The process as claimed in one or more of claims 1 to 5, wherein an organometallic compound of the formula MgXR, LiR, AlX₂R, AlXR₂, AlR₃ or Al₂Cl₃R₃ is used.

7. The process as claimed in one or more of claims 1 to 6, wherein an organometallic compound of the formula MgXR, LiR or AlR₃ is used.

8. The process as claimed in one or more of claims 1 to 7, wherein, as an inert solvent, an aromatic hydrocarbon having 6 to 14 C atoms, a trialkylamine having 1 to 18 C atoms per alkyl radical, where the alkyl radical may contain alkoxy groups having 1 to 4 C atoms as substituents, an aliphatic ether having 1 to 6 C atoms per alkyl radical, a cycloaliphatic ether having 4 to 6 carbon atoms in the ring, an alkyl ether of polyhydric alcohols having 2 to 6 C atoms in the alcohol radical and 1 to 4 C atoms per alkyl radical, an alkyl ether of polyethylene glycol having 2 to 22 ethylene glycol units and 1 to 4 C atoms per alkyl radical or a mixture of these solvents is used.

9. The process as claimed in one or more of claims 1 to 8, wherein, as inert solvent, an aliphatic ether having 1 to 6 C atoms per alkyl radical, a cycloaliphatic ether having 4 to 6 C atoms in the ring, an alkyl ether of polyhydric alcohols having 2 to 6 C atoms in the alcohol radical and 1 to 4 C atoms per alkyl radical or a mixture of these solvents is used.

10. The process as claimed in one or more of claims 1 to 9, wherein the reaction is carried out at from -70 to +100°C.

11. The process as claimed in one or more of claims 1 to 10, wherein the acid used to treat the reaction product is a mineral acid or an aliphatic carboxylic acid having 1 to 4 carbon atoms.

12. The process as claimed in one or more of claims 1 to 11, wherein the acid used to treat the reaction product is sulfuric acid.

13. The process as claimed in one or more of claims 1 to 12, wherein the decarboxylation is carried out in the presence of an inert solvent or a base or in the presence of an inert solvent and a base.

## Revendications

1. Procédé de préparation d'acides trifluorobenzoïques de formule 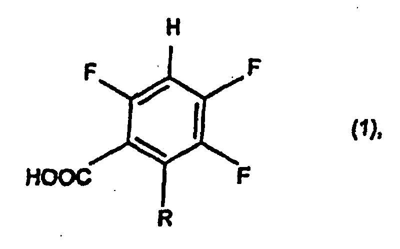 dans laquelle R représente un reste alkyle linéaire ou ramifié de 1 à 6 atomes de carbone, un reste phényle non substitué, un reste phényle substitué contenant un ou deux groupes alkyle ou alcoxy ayant chacun 1 à 4 atomes de carbone, ou un reste araliphatique de 7 à 12 atomes de carbone, **caractérisé en ce que** l'on fait réagir un composé de formule 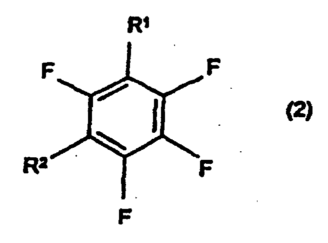 dans laquelle R¹ et R² sont identiques ou différents et représentent -CN, -COOR³, où R³ représente H, Li, Na, K, MgCl, MgBr, MgI, 1/2 Mg, 1/2 Ca ou un reste alkyle de 1 à 6 atomes de carbone, ou -CONR⁴R⁵, où R⁴ et R⁵ sont identiques ou différents et représentent un reste alkyle de 1 à 6 atomes de carbone, avec au moins un composé organométallique de formule CuR, CuLiR₂, MgXR, ZnR₂, LiR, AlX₂R, AlR₃ ou Al₂Cl₃R₃, R ayant la définition donnée pour la formule (1) et X étant Cl, Br ou I, en présence d'un solvant inerte à une température de -80 à +150°C, on traite le produit de réaction en présence d'eau, en l'absence ou en présence d'un acide, à une température de 0 à 250°C, et on décarboxyle l'acide trifluoroisophtalique ainsi formé de formule 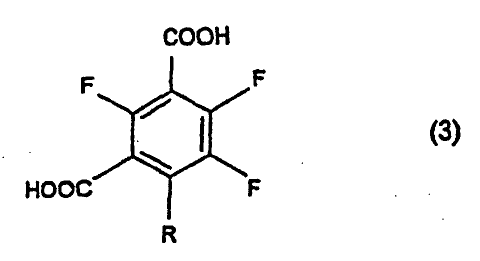 à une température de 80 à 280°C.

2. Procédé selon la revendication 1, **caractérisé en ce que** l'on utilise un composé de formule (2) dans lequel R¹ et R² sont identiques ou différents et représentent -CN ou -COOR³, R³ étant Na, K, MgCl ou MgBr.

3. Procédé selon la revendication 1 ou 2, **caractérisé en ce que** l'on utilise un composé de formule (2) dans lequel R¹ et R² sont identiques et représentent -CN, -COONa ou -COOMgCl.

4. Procédé selon l'une ou plusieurs des revendications 1 à 3, **caractérisé en ce que** l'on utilise un composé de formule (2) dans lequel R¹ et R² sont identiques et représentent -COOLi, -COOMgCl, -COOMgBr ou -COOMgI, préparé par réaction d'acide tétrafluoroisophtalique dans un solvant inerte avec un composé organométallique LiR⁶, MgClR⁶ ou MgIR⁶, dans lequel R⁶ représente un reste alkyle linéaire ou ramifié de 1 à 6 atomes de carbone, un reste phényle non substitué, un reste phényle substitué contenant un ou deux groupes alkyle ou alcoxy ayant chacun 1 à 4 atomes de carbone, ou un reste araliphatique de 7 à 12 atomes de carbone.

5. Procédé selon l'une ou plusieurs des revendications 1 à 4, **caractérisé en ce que** l'on utilise un composé de formule (2) dans lequel R¹ et R² sont identiques et représentent -COOMgCl, -COOMgBr ou -COOMgI, préparé par réaction d'acide tétrafluoroisophtalique dans un solvant inerte avec un composé organométallique MgClR⁶, MgBrR⁶, ou MgIR6, dans lequel R⁶ représente un reste o-tolyle.

6. Procédé selon l'une ou plusieurs des revendications 1 à 5, **caractérisé en ce que** l'on utilise un composé organométallique de formule MgXR, LiR, AlX₂R, AlXR₂, AlR₃ ou Al₂Cl₃R₃.

7. Procédé selon l'une ou plusieurs des revendications 1 à 6, **caractérisé en ce que** l'on utilise un composé organométallique de formule MgXR, LiR ou AlR₃.

8. Procédé selon l'une ou plusieurs des revendications 1 à 7, **caractérisé en ce que** l'on utilise comme solvant inerte un hydrocarbure aromatique de 6 à 14 atomes de carbone, une trialkylamine ayant 1 à 18 atomes de carbone dans chaque reste alkyle, le reste alkyle pouvant contenir des groupes alcoxy de 1 à 4 atomes de carbone comme substituant, un éther aliphatique ayant 1 à 6 atomes de carbone dans chaque groupe alkyle, un éther cycloaliphatique ayant 4 à 6 atomes de carbone cycliques, un éther alkylique de polyalcool ayant 2 à 6 atomes de carbone dans le reste alcool et 1 à 4 atomes de carbone dans chaque reste alkyle, un éther alkylique de polyéthylèneglycol ayant 2 à 22 unités d'éthylèneglycol et 1 à 4 atomes de carbone dans chaque reste alkyle, ou un mélange de ces solvants.

9. Procédé selon l'une ou plusieurs des revendications 1 à 8, **caractérisé en ce que** l'on utilise comme solvant inerte un éther aliphatique ayant 1 à 6 atomes de carbone dans chaque reste alkyle, un éther cycloaliphatique ayant 4 à 6 atomes de carbone cycliques, un éther alkylique de polyalcool ayant 2 à 6 atomes de carbone dans le reste alcool et 1 à 4 atomes de carbone dans chaque reste alkyle, ou un mélange de ces solvants.

10. Procédé selon l'une ou plusieurs des revendications 1 à 9, **caractérisé en ce que** l'on effectue la réaction à une température de -70 à +100°C.

11. Procédé selon l'une ou plusieurs des revendications 1 à 10, **caractérisé en ce que** l'on traite le produit de réaction avec, comme acide, un acide inorganique ou un acide carboxylique aliphatique de 1 à 4 atomes de carbone.

12. Procédé selon l'une ou plusieurs des revendications 1 à 11, **caractérisé en ce que** l'on traite le produit de réaction avec, comme acide, de l'acide sulfurique.

13. Procédé selon l'une ou plusieurs des revendications 1 à 12, **caractérisé en ce que** l'on effectue la décarboxylation en présence d'un solvant inerte ou d'une base, ou en présence d'un solvant inerte et d'une base.
